# EUROPEAN PATENT APPLICATION

(11) **EP 4 763 251 A2**
(43) Date of publication of application: **24.06.2026**
(21) Application number: 26175437.8
(22) Date of filing: 18.12.2020
(51) Int. Cl.: A61M 5/32

(54) **SYRINGE APPARATUS**

(30) Priority: 20.12.2019 GB 201919085
(62) Divisional of application: 20839283.7
(71) Applicant: Owen Mumford Ltd., Oxfordshire OX7 5XP (GB)
(72) Inventor: HUTT, Rosie, Chipping Norton, OX7 5XP (GB); DOBSON, Matthew John, Chipping Norton, OX7 5XP (GB)
(74) Representative: Marks & Clerk LLP

(57) **Abstract**

An apparatus for use with an auto-injector configured to receive a syringe, the apparatus comprising: a firing assembly comprising a rearward portion, a plunger rod moveable forward relative to the rearward portion, a plunger rod driver configured to drive the plunger rod forward relative to the rearward portion and a retainer engaged with the plunger rod and/or the rearward portion to prevent forward movement of the plunger rod relative to the rearward portion, wherein the firing assembly is configured for insertion into the auto-injector such that the plunger rod is received within a barrel of the syringe, and wherein the retainer is configured to engage an axial lock for axially fixing a position of the retainer relative to the auto-injector when a forward end of the plunger rod is at a particular position relative to a bung located within the barrel.

## Description

### Technical field

The invention relates to auto-injectors and, more specifically, to an apparatus for use with auto-injectors.

### Background

An auto-injector is a device for receiving a syringe and for driving a syringe plunger into a barrel of the syringe, typically for drug delivery, without any force being applied by the user. Typically, an auto-injector includes a plunger driver, often comprising a spring that is arranged to provide a force to drive the syringe plunger into the barrel. The plunger driver is typically activated by operation of a release mechanism on the auto-injector, such as a button or through pressure applied to an injection site via the auto-injector.

### Summary

According to the invention in a first aspect, there is provided an apparatus for use with an auto-injector configured to receive a syringe, the apparatus comprising: a firing assembly comprising a rearward portion, a plunger rod moveable forward relative to the rearward portion and a plunger rod driver configured to drive the plunger rod forward relative to the rearward portion, an insertion driver configured to drive the syringe forwards on an insertion stroke for inserting a needle of the syringe into an injection site, wherein the firing assembly further comprises a retainer engaged with the plunger rod and/or the rearward portion and configured to prevent forward movement of the plunger rod relative to the rearward portion, and to disengage from the firing assembly at a point on the insertion stroke.

Optionally, the insertion driver is further configured to drive at least the plunger rod, the plunger rod driver and the rearward portion forwards on the insertion stroke.

Optionally, the disengagement of the retainer is caused by relative axial movement between the retainer and the plunger rod, the plunger rod driver and the rearward portion.

Optionally, the retainer is axially fixed relative to a housing of the auto-injector.

Optionally, the plunger rod driver biases the plunger rod forwards relative to the rearward portion.

Optionally, one of the rearward portion and the plunger rod comprises an angled surface configured to interact with a corresponding feature on the other of the rearward portion and plunger rod under the bias of the plunger rod driver for relative rotation between the rearward portion and plunger rod, wherein the retainer is configured to prevent relative rotation between the rearward portion and plunger rod.

Optionally, the retainer comprises a prong configured to abut a surface of the plunger rod to prevent relative rotation between the rearward portion and plunger rod.

Optionally, forward movement of the firing assembly on the insertion stroke draws the prong out of abutment with the surface of the plunger rod.

Optionally, the axial length of the prong defines the point on the insertion stroke at which the retainer disengages from the firing assembly.

Optionally, the point on the insertion stroke at which the retainer disengages from the firing assembly is at substantially the end of the insertion stroke.

According to the invention in a further aspect, there is provided an auto-injector sub-assembly comprising an apparatus and a housing, the auto-injector sub-assembly comprising: a firing assembly comprising a rearward portion, a plunger rod moveable forward relative to the rearward portion and a plunger rod driver configured to drive the plunger rod forward relative to the rearward portion, the firing assembly insertable within the housing, an insertion driver configured to drive the syringe forwards on an insertion stroke for inserting a needle of the syringe into an injection site, wherein the firing assembly further comprises a retainer engaged with the plunger rod and/or the rearward portion and configured to prevent forward movement of the plunger rod relative to the rearward portion, and to disengage from the firing assembly at a point on the insertion stroke.

According to the invention in a further aspect, there is provided an apparatus for use with an auto-injector configured to receive a syringe, the apparatus comprising: a firing assembly comprising a rearward portion, a plunger rod moveable forward relative to the rearward portion, a plunger rod driver configured to drive the plunger rod forward relative to the rearward portion and a retainer engaged with the plunger rod and/or the rearward portion to prevent forward movement of the plunger rod relative to the rearward portion, wherein the firing assembly is configured for insertion into the auto-injector such that the plunger rod is received within a barrel of the syringe, and wherein the retainer is configured to engage an axial lock for axially fixing a position of the retainer relative to the auto-injector when a forward end of the plunger rod is at a particular position relative to a bung located within the barrel.

Optionally, the apparatus further comprises an actuator coupled to the firing assembly and configured to be received in a housing of the auto-injector for insertion of the firing assembly into the auto-injector, wherein the actuator comprises the axial lock.

Optionally, the retainer is configured to engage the axial lock on relative rotation between the actuator and the retainer.

Optionally, the actuator comprises a plurality of locking features, and wherein relative rotation between the actuator and the retainer causes engagement between a retaining feature of the retainer and one of the plurality of locking features.

Optionally, one of the actuator and the firing assembly comprises a ramped surface configured to interact with a corresponding feature on the other of the actuator and firing assembly to cause the relative rotation between the retainer and the actuator.

Optionally, the actuator comprises a sleeve configured to receive the firing assembly therein.

Optionally, the actuator further comprises an end cap configured to abut a surface of the housing to fix the axial position of the actuator relative to the housing.

Optionally, the retainer is configured to engage the axial lock when the end cap abuts the surface of the auto-injector.

Optionally, the apparatus further comprises an insertion driver configured to drive the syringe, the plunger rod, the plunger rod driver and the rearward portion forwards on an insertion stroke for inserting a needle of the syringe into an injection site.

Optionally, the axial lock is configured to prevent forward movement of the retainer such that the forward movement of the syringe, the plunger rod, the plunger rod driver and the rearward portion forwards on the insertion stroke disengages the retainer from the plunger rod and/or the rearward portion.

According to the invention in a further aspect, there is provided an auto-injector sub-assembly comprising an apparatus according to any of claims 12 to 22, and further comprising a housing configured to receive the apparatus.

According to the invention in a further aspect, there is provided an auto-injector comprising the apparatus of any of claims 1 to 10 and claims 12 to 21, and/or the auto-injector sub-assembly of claims 11 or 22.

Optionally, the auto-injector further comprises a syringe comprising a barrel and a bung located therein.

According to the invention in a further aspect, there is provided a method of assembling an apparatus for use with an auto-injector, the method comprising: at least partially inserting a firing assembly into a barrel of a syringe, the firing assembly comprising a rearward portion, a plunger rod moveable forward relative to the rearward portion, a plunger rod driver configured to drive the plunger rod forward relative to the rearward portion and a retainer engaged with the plunger rod and/or the rearward portion to prevent forward movement of the plunger rod relative to the rearward portion; and engaging the retainer with an axial lock for axially fixing a position of the retainer relative to the auto-injector when a forward end of the plunger rod is at a particular position relative to a bung located within the barrel.

Optionally, the apparatus may further comprise an actuator coupled to the firing assembly, wherein at least partially inserting the firing assembly into the barrel of the syringe comprises at least partially inserting the actuator into the barrel of the syringe.

Optionally, the actuator comprises the axial lock, and engaging the retainer with the axial lock comprises causing relative rotation between the actuator and the retainer.

Optionally, wherein one of the actuator and the firing assembly comprises a ramped surface configured to interact with a corresponding feature on the other of the actuator and firing assembly to cause the relative rotation between the retainer and the actuator.

Optionally, inserting the actuator into the housing comprises moving the actuator forwards within the housing until an end cap of the actuator abuts a surface of the housing.

Optionally, the retainer is configured to engage the axial lock when the end cap abuts the surface of the auto-injector.

### Brief description of drawings

Figure 1 shows an isometric view of an exemplary apparatus;
Figure 2a shows an exploded view of an exemplary firing assembly;
Figure 2b shows an isometric view of the exemplary firing assembly of Figure 2a;
Figure 2c shows a schematic view of an exemplary firing assembly;
Figure 3 shows an isometric view of an exemplary cartridge;
Figures 4a is a plan view of the exemplary apparatus in a first position, and Figure 4b is an isometric view of the exemplary apparatus in the first position;
Figures 5a is a plan view of the exemplary apparatus in a second position, and Figure 5b is an isometric view of the exemplary apparatus in the second position; and
Figures 6a is a plan view of the exemplary apparatus in a third position, and Figure 6b is an isometric view of the exemplary apparatus in the third position.

### Description

Generally, disclosed herein are apparatus for use with auto-injectors. The exemplary apparatus may comprise firing assemblies configured to be actuated under the force of an insertion driver on an insertion stroke for inserting a needle of the auto-injector into an injection sire. Actuation of the firing assembly may cause release of a plunger rod of the firing assembly to drive a bung of a syringe within the auto-injector forwards to dispense a substance therefrom. In exemplary apparatus, actuation of the firing assembly may occur at substantially the end of the insertion stroke. In such arrangements, separate actuation of the firing assembly by the user is not required. The user simply needs to actuate the device to release the insertion driver, and then the insertion driver automatically actuates the firing assembly at a point on the insertion stroke.

Exemplary firing assemblies may comprise a rearward portion, a plunger rod and a plunger rod driver. The plunger rod may be moveable forwards with respect to the rearward portion under the force of the plunger rod driver. In exemplary arrangements, the firing assembly may comprise a retainer configured to prevent forward movement of the plunger rod with respect to the rearward portion when engaged with at least one of the plunger rod and/or rearward portion. On disengagement of the retainer, the plunger rod may be free to move forward relative to the rearward portion under the force of the plunger rod driver to dispense the substance from the syringe of the auto-injector. In such arrangements, the retainer may be configured to disengage from the plunger rod and/or rearward portion at a point on the insertion stroke, which may be substantially the end of the insertion stroke.

Disengagement of the retainer from the plunger rod and/or rearward portion may be caused by relative axial movement between the retainer and the plunger rod and/or rearward portion. In exemplary arrangements, insertion of the apparatus into a housing of the auto-injector during assembly of the auto-injector may cause the retainer to engage with an axial lock to fix the axial position of the retainer relative to the housing. The plunger rod, plunger rod driver and rearward portion may be configured to move forwards on the insertion stroke under the force of the insertion driver. As such, forward movement of the plunger rod and/or rearward portion under the force of the insertion driver may cause the relative axial movement between the retainer and the plunger rod and/or rearward portion to disengage the retainer.

Throughout the specification, the term "forward" encompasses the end of the auto-injector from which the medicament is delivered. In other words, the forward end of the auto-injector is the end proximal to an injection site during use (i.e. the needle end). As such, "forward movement" refers to movement of an apparatus/component towards the needle end, and a "forward end" of an apparatus/component refers to the end of the component/apparatus closest to the needle end of the auto-injector when the apparatus/component is received therein. The terms "rear" or "rearward" mean distant or remote from the injection site during use.

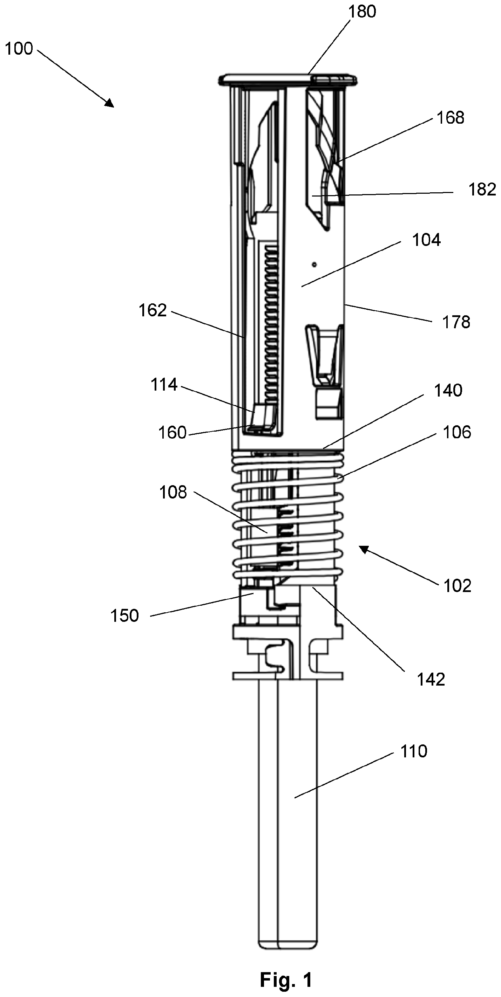

Figure 1 shows an apparatus 100 for use with an auto-injector. The apparatus 100 may be configured for insertion into a housing of an auto-injector. The apparatus 100 comprises a firing assembly 102, an actuator 104 and an insertion driver 106.

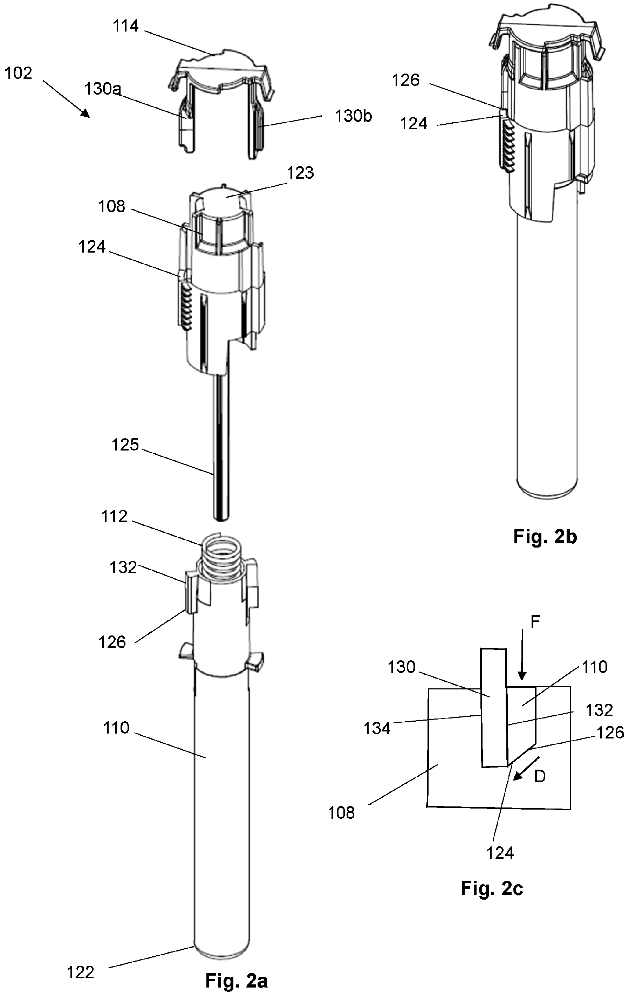

Figure 2a shows an exploded view of the firing assembly 102 and Figure 2b shows the exemplary firing assembly 102 in isolation from the actuator 104 and the insertion driver 106. The firing assembly 102 comprises a rearward portion 108, a plunger rod 110, a plunger rod driver 112 and a retainer 114. The firing assembly is described below, with reference to Figures 2a and 2b.

In the arrangement shown in Figures 2a and 2b, the plunger rod 110 is moveable relative to the rearward portion 108. The plunger rod 110 may be moveable forwards relative to the rearward portion 108. In the exemplary firing assembly 102, the plunger rod driver 112 is configured to drive the plunger rod 110 forward relative to the rearward portion 108. The retainer 114 is configured for engagement with the plunger rod 110 and/or the rearward portion 108 to prevent forward movement of the plunger rod 110 relative to the rearward portion.

In the exemplary arrangement shown in Figures 2a and 2b, the plunger rod driver 112 is disposed between the rearward portion 108 and the plunger rod 110. The plunger rod driver 112 may be configured to bias the plunger rod 110 forwards relative to the rearward portion 108. In the exemplary firing assembly 102 of Figures 2a and 2b, the plunger rod driver 112 comprises a compression spring. In the context of the specification the term "driver" is used to encompass substantially any component capable of exerting a force on the plunger rod 110. The skilled person will therefore appreciate that in alternative arrangements, the plunger rod driver 112 may comprise alternative biasing members, such as extension or tension springs.

The retainer 114 may be configured such that engagement with the plunger rod 110 and/or the rearward portion 108 prevents axial separation of the plunger rod 110 and the rearward portion 108. In such arrangements, the plunger rod driver 112 may be held in a primed, or energised, state between the rearward portion 108 and the plunger rod 110. The plunger rod driver 112 may be configured to bias the plunger rod 110 forwards relative to the rearward portion 108 on release of the plunger rod driver 112 from the primed state. In the exemplary arrangement shown in Figures 2a and 2b, the plunger rod driver 112 may be released from the primed state when the retainer 114 is disengaged from the plunger rod 110 and/or rearward portion 108 to allow forward movement of the plunger rod 110 relative to the rearward portion 108 under the bias of the plunger rod driver 112.

In the exemplary arrangement shown in Figures 2a and 2b, the plunger rod driver 112 is held in the primed state between a surface of the plunger rod and a reaction surface of the rearward portion 114. In the exemplary arrangement Figures 2a and 2b, the plunger rod 110 comprises a bore 120 within which the plunger rod driver 112 is received. A forward end of the plunger rod driver 112 may abut an internal surface of the plunger rod 110. The internal surface of the plunger rod 110 may be proximal to a forward end 122 of the plunger rod 110. A rearward end of the plunger rod driver 112 may abut an internal surface of the rearward portion 108. The internal surface of the rearward portion 108 may be proximal to a rearward end 123 of the rearward portion 108.

The rearward portion may comprise a pin 125. The pin 125 may extend axially forwards from a rearward surface of the rearward portion 123 (for example, the internal surface of the rearward portion 108 described above). In the exemplary arrangement shown in Figures 2a and 2b, the plunger rod driver 112 comprises a compression spring. In such arrangements, the pin 125 may be received within a central bore of the compression spring. The pin 125 may extend along substantially the whole length of the compression spring in its primed state. The pin 125 may be configured to prevent buckling of the compression spring when the compression spring is placed into the primed state and/or act as a guide when the compression spring is released from the primed state and expands.

As will be described below, in exemplary arrangements, the rearward portion 108 may be axially fixed relative to a housing of the auto-injector when the firing assembly 102 is fully inserted into the housing. In such arrangements, when the plunger rod driver 112 is released from the primed state, the plunger rod 110 is driven forwards with respect to the rearward portion 108 and rearward movement of the rearward portion 108 under the force of the plunger rod driver 112 is prevented.

The plunger rod 110 may be telescopically received within the rearward portion 108. The rearward portion 108 may comprise an angled surface 124 configured to interact with a corresponding feature 126 (or plunger rod follower) on the plunger rod 110. Interaction between the angled surface 124 and the corresponding feature 126 may cause relative rotation between the rearward portion 108 and the plunger rod 110. In exemplary arrangements, the corresponding feature 126 of the plunger rod 110 is configured to travel along the angled surface 124 of the rearward portion 108 to cause the relative rotation. The angled surface 124 of the rearward portion 108 may therefore translates axial force applied to the plunger rod 110 into rotational movement of the plunger rod 110 relative to the rearward portion 108. In the exemplary arrangement shown in Figures 2a and 2b, the plunger rod 110 comprises two corresponding features and the rearward portion 108 comprises two angled surfaces. The skilled person will appreciate however, that in alternative arrangements, the plunger rod 110 and the rearward portion 108 may comprise substantially any number of corresponding features and angled surfaces.

In the example shown in Figures 2a and 2b, the corresponding feature 126 of the plunger rod 110 comprises a corresponding angled surface. However the skilled person will appreciate that in alternative arrangements, the corresponding feature 126 may comprise a peg or a lug, or any other feature capable of interacting with the angled surface to translate axial force into rotational force. The skilled person will further appreciate that in alternative arrangements, the plunger rod 110 may comprise the angled surface 124 and the rearward portion 108 may comprise the corresponding feature 126.

In exemplary arrangements, the plunger rod driver 112 biases the corresponding feature 126 of the plunger rod 110 into engagement with the angled surface 124 of the rearward portion 108.

In the examples shown in Figures 2a and 2b, the retainer 114 is configured to prevent forward movement of the plunger rod 110 relative to the rearward portion 108. The retainer may be configured to prevent forward movement of the plunger rod 110 relative to the rearward portion by preventing separation of the plunger rod 110 and the rearward portion 108. As such, the plunger rod driver 112 is held in a primed, or energised, state. In the exemplary arrangement of Figures 2a and 2b, the plunger rod driver 112 is held in a primed state between the internal surfaces of the plunger rod 110 and the rearward portion 108.

In the examples shown, the retainer 114 may be configured to prevent forward movement of the plunger rod 110 relative to the rearward portion 108 by preventing relative rotation between the plunger rod 110 and the rearward portion 108. In such arrangements, the retainer 114 may be configured to prevent travel of the corresponding feature 126 on the plunger rod 110 along the angled surface 124 of the rearward portion 108.

The retainer 114 may comprise at least one prong 130 configured to resist rotation of the plunger rod 110 with respect to the rearward portion 108. In the exemplary arrangement shown in Figures 2a and 2b, the retainer comprises a pair of opposed prongs 130a, 130b, however the skilled person will appreciate that alternative arrangements may comprise substantially any number of prongs.

Figure 2c shows a schematic representation of the interaction between the prong 130, the plunger rod 110 and the rearward portion 108 according to the exemplary arrangement of Figures 2a and 2b. The plunger rod 110 is biased forwards under the force of the plunger rod driver 112 (shown as arrow F). The corresponding feature 126 (or plunger rod follower) of the plunger rod 110 abuts the angled surface 124 of the rearward portion 108, such that the corresponding feature 126 is biased to travel along the angled surface 124 of the rearward portion 108 under the force F of the plunger rod driver 112. The force F therefore acts to rotate the plunger rod 110 relative to the rearward portion 108 in direction D. Movement of the corresponding feature 126 of the plunger rod 110 along the angled surface 124 of the rearward portion is prevented by the prong 130.

The prong 130 is configured to abut surfaces 132 and 134 of the plunger rod 110 and the rearward portion 108 respectively. As can be seen in Figure 2c, the plunger rod driver 112 wants to bias the plunger rod 110 in direction D, due to the engagement of the corresponding feature 126 and the angled surface 124. However, movement of the corresponding feature 126 along the angled surface 124 in direction D is prevented by the prong 130. The surface 132 of the plunger rod is pushed against the prong 130 in direction D under the force of the plunger rod driver 112, however movement of the plunger rod 110 and the prong 130 in direction D is prevented by the engagement of the prong 13- with the surface 134 of the rearward portion 108.

In the exemplary arrangement shown in Figures 2a and 2b, the retainer 114 may be disengageable from the plunger rod 110 and/or rearward portion 108 to allow forward movement of the plunger rod 110 relative to the rearward portion 108. In such arrangements, the retainer 114 may be disengageable with the plunger rod 110 and/or rearward portion 108 on relative axial movement between the retainer and the plunger rod 110 and/or the rearward portion 108 (and as such, the plunge rod driver 112).

In the exemplary arrangement shown in Figures 2a and 2b, axial movement of the plunger rod 110 and/or the rearward portion 108 relative to the retainer 114 draws the prong 130 out of abutment with the surfaces 132, 134 of the plunger rod 110 and the rearward portion 108. The skilled person will appreciate that the extent of relative axial movement required to draw the prong 130 out of engagement with the surfaces 132, 134 may be defined by an axial length of the prong 130. Drawing the prong 130 out of engagement with the surfaces 132, 134 may allow relative rotation between the plunger rod 110 and the rearward portion 108, and as such forward movement of the plunger rod 110 relative to the rearward portion 108 under the force of the plunger rod driver 112.

The insertion driver 106 will now be described with reference to Figure 1. The insertion driver 106 may be configured to drive a syringe forwards within an auto-injector on an insertion stroke for inserting a needle of the syringe into an injection site.

In the exemplary arrangement shown in Figure 1, the insertion driver 106 may be further configured to drive one or more components of the firing assembly 102 forwards on the insertion stroke. In exemplary arrangements, the one or more components may comprise at least the rearward portion 108, the plunger rod 110 and the plunger rod driver 112.

In the example shown in Figure 1, the insertion driver 106 is disposed between the actuator 104 and a reaction surface 140 of the firing assembly 102. The insertion driver 106 of Figure 1 comprises a compression spring. However, the skilled person will appreciate that in alternative arrangements, alternative drivers may be utilised to exert a forward force on the one or more components of the firing assembly 103. For example, the insertion driver 106 may comprise extension or tension springs.

In the exemplary arrangement of Figure 1, the insertion driver 106 is disposed between the reaction surface 140 of the actuator 104 and a surface 142 of the firing assembly 102. As will be described in more detail below, an axial position of the actuator 104 relative to the auto-injector may be fixed such that release of the insertion driver 106 drives at least the rearward portion 108, the plunger rod 110 and the plunger rod driver 112 forwards on the insertion stroke, without causing rearward movement of the actuator 104.

In the example shown in Figure 1, the firing assembly 102 further comprises a cartridge 150. The term "cartridge" may encompass any sleeve, collar or other component capable of receiving the firing assembly. For example, a sleeve, collar or other component configured to telescopically receive the firing assembly such that the firing assembly is moveable therein.

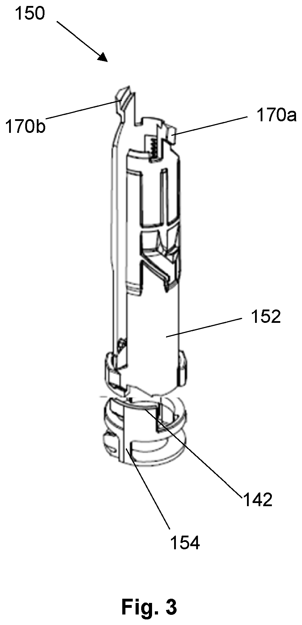

The cartridge 150 may be configured to receive the firing assembly 102 therein. In the exemplary arrangement of Figure 1, the cartridge 150 telescopically received the firing assembly 102. The firing assembly 102 may be coupled to the cartridge 150 such that axial movement of the cartridge 150 causes corresponding axial movement of the firing assembly 102. In exemplary arrangements the cartridge 150 may comprise the surface 142. As such, in exemplary arrangements the insertion driver 106 may be disposed between the reaction surface 140 of the actuator and the surface 142 of the cartridge 150. The cartridge 150 is shown in isolation in Figure 3.

As can be seen in Figure 3, the exemplary cartridge 150 may comprise a first portion 152 and a second portion 154. However, the skilled person will appreciate that in alternative arrangements, the cartridge 150 may comprise a single portion.

In the exemplary arrangement shown in Figure 1, the firing assembly 103 may be coupled to the cartridge 150 via the rearward portion 108. The rearward portion 108 may comprise locking features configured to engage corresponding locking features on the cartridge 150. As such, the insertion driver 106 may be configured to drive one or more components of the firing assembly 103 forwards. In exemplary arrangements, the one or more components of the firing assembly comprise at least the rearward portion 108, the plunger rod 110 and the plunger rod driver 112.

In alternative arrangements, the rearward portion 108 may comprise one or more of the features of the cartridge 150. In such arrangements there may be no separate cartridge component.

In exemplary arrangements, the retainer 114 may be configured to engage an axial lock for axially fixing a position of the retainer 114 relative to a housing of the an auto-injector. The retainer 114 may comprise at least one retaining feature 160 configured to engage the axial lock. In the examples shown, the retaining feature 160 comprises radially extending projections, however the skilled person will appreciate that alternative features may be utilised to engage the axial lock.

In the exemplary apparatus 100 shown in Figure 1, the actuator 104 comprises the axial lock 162. In such examples, engagement of the retaining feature 160 with the axial lock axially fixes the position of the retainer 114 relative to the actuator 104. As will be described in more detail below, the position of the actuator 104 may be axially fixed with respect to a housing of an auto-injector, and as such, the engagement of the retaining feature 160 with the axial lock 162 axially fixes the position of the retainer 114 relative to the housing of the auto-injector.

The axial lock 162 may comprise a plurality of locking features. The plurality of locking features may be axially spaced along at least part of a length of the actuator 104. In the exemplary actuator 104 shown in Figure 1, the plurality of locking features are axially spaced over substantially the entire length of the actuator 104.

The retaining feature 160 of the retainer 114 may be configured to engage one of the plurality of locking features of the axial lock 162 to fix the position of the retainer 114 relative to the actuator 104. The skilled person will appreciate therefore that the retainer 114 may be fixable to the actuator 104 at a plurality of different points along the length of the actuator. Although the exemplary locking features in the arrangement shown in Figure 1 provide a plurality of discrete positions along the length of the actuator 104 to which the retainer 114 may be fixed, continuous locking arrangements may alternatively be provided such that the retainer 114 may be fixed to the actuator 102 at substantially any point along the length of the actuator 104.

The retainer 114 may be configured to engage the axial lock 162 on relative rotation between the actuator 104 and the retainer 114. In such arrangements, relative rotation between the actuator 104 and the retainer 114 may cause engagement of the retaining feature 160 of the retainer 114 with one of the plurality of locking features of the axial lock 162.

In the exemplary actuator 104, the plurality of locking features of the axial lock 162 comprise teeth. The teeth may be arranged in a linear track which extends along at least part of a length of the actuator 104. In the exemplary arrangement of Figure 1, the teeth may be located on an internal surface of the actuator 104. The teeth may be rearwardly angled and configured to engage a correspondingly angled surface of the retaining feature 160 on the retainer 114.

The firing assembly 102 may be coupled to the actuator 104. In the exemplary arrangement shown in Figure 1, the firing assembly 102 is telescopically received within the actuator 104. The actuator may comprise a ramped surface 168 configured to interact with a corresponding feature on the firing assembly 102 to cause the relative rotation between the retainer 114 and the actuator 104 to engage the retainer with the axial lock 162.

In the exemplary arrangement shown in Figure 1, the cartridge 150 comprises the corresponding feature 170 (or cartridge follower), visible in Figure 3. As described above, the rearward portion 108, the plunger rod 110, the plunger rod driver 112 and retainer 114 are coupled to the cartridge 150. As such, rotation of the cartridge 150 relative to the actuator 104 causes rotation of the rearward portion 108, the plunger rod 110, the plunger rod driver 112 and retainer 114 relative to the actuator 104.

In the exemplary arrangement of Figure 1, the actuator 104 comprises a sleeve 178. The actuator 104 may further comprise an end cap 180. The end cap 180 may be configured to abut a rearward face of the housing of the auto-injector device when the apparatus 100 is fully inserted therein.

In the exemplary arrangement of Figure 1, the sleeve 178 extends from the end cap 180. The sleeve 178 may be configured to receive the firing assembly 102 therein. In the arrangement of Figure 1, the sleeve 178 is configured to receive the cartridge 150 therein. In such arrangements, the sleeve 178 may comprise the ramped surface 168.

The actuator 104 may further comprise a drive track 182. The drive track 182 may comprise an axial section to allow axial movement of the corresponding feature 170 of the cartridge 150 therein. The corresponding feature 170 of the cartridge may be configured to travel along the drive track to allow relative axial movement between the actuator 104 and the drive track 182 under the bias of the insertion driver 106.

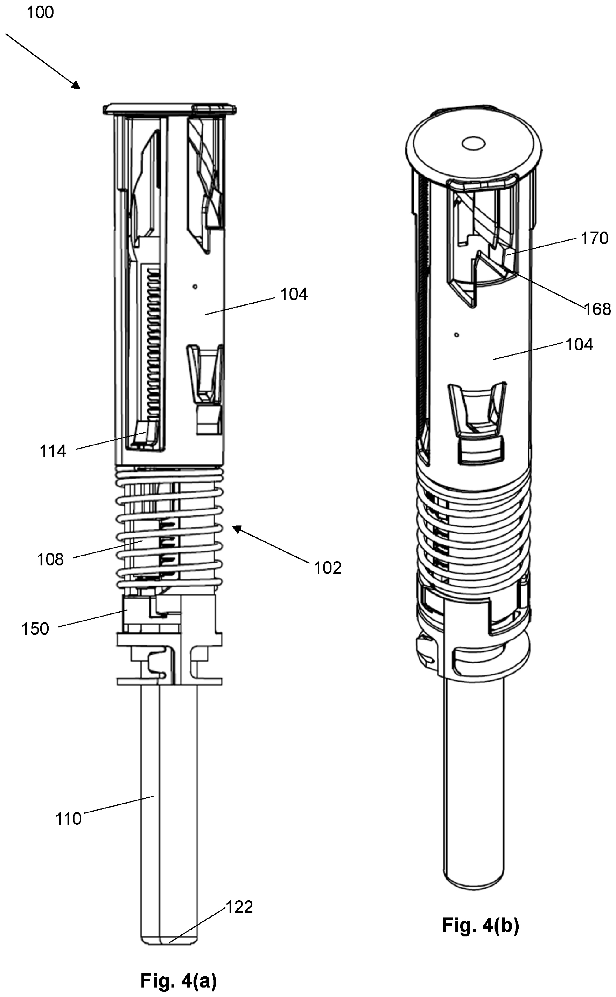

Operation of apparatus 100 will now be described with reference to Figures 4a-6b.

Figures 4a and 4b show the apparatus 100 in an initial position. In the initial position, the retainer 114 is engaged with the plunger rod 110 and/or rearward portion 108 to prevent forward movement of the plunger rod 110 relative to the rearward portion 108, as described above. At this point, in exemplary arrangements, the rearward portion 108 may be decoupled from the cartridge 150 such that the rearward portion 108, the plunger rod 110, the plunger rod driver 112 and the retainer 114 are moveable axially with respect to the cartridge 150. In alternative arrangements, the rearward portion 108 may be coupled to the cartridge 150 in the initial position.

The corresponding feature 170 of the cartridge 150 may be engaged with the ramped surface 168 of the actuator 104. As shown in Figure 4b, the corresponding feature 170 may be located at the forwardmost point of the ramped surface 168.

The firing assembly 102 may be inserted into a housing of an auto-injector. The auto-injector housing may have received a syringe comprising a barrel and bung. In exemplary auto-injectors, the syringe may be received and held proximal to a forward end of the auto-injector housing.

The firing assembly 102 may be moved forwards within the housing of the auto-injector such that the plunger rod 110 is received within the barrel of the syringe located within the auto-injector housing. In exemplary arrangements, an axial force may be applied to the actuator 104 to insert the firing assembly 102 into the housing of the auto-injector.

At a point during the forward movement of the firing assembly 102 within the housing, the forward end 122 of the plunger rod 110 may contact the bung located within the barrel of the syringe within the auto-injector. The skilled person will appreciate that the syringe may be a pre-filled syringe and in such arrangements, the point at which the plunger rod 110 contacts the bung will depend on the fill volume of the pre-filled syringe.

Once the plunger rod 110 contacts the bung, further forward movement of the rearward portion 108, the plunger rod 110, the plunger rod driver 112 and the retainer 114 is prevented. This is because as described above, the rearward portion 108 and the plunger rod driver 112 are coupled to the plunger rod 110 by the retainer 114. As such, further force applied to the actuator 104 causes relative axial movement between the cartridge 150 and at least the rearward portion 108, the plunger rod 110, the plunger rod driver 112 and the retainer 114.

In the exemplary arrangement of Figures 4a and 4b, since the actuator 104 and the cartridge 150 are coupled, further forward movement of the actuator 104 under the axial force causes further forward movement of the cartridge 150. The cartridge 150 therefore continues to move forwards within the housing, while the rearward portion 108, the plunger rod 110, the plunger rod driver 112 and the retainer 114 remain in substantially the same axial position relative to the housing. As such, more of the length of the plunger rod 110 is received within the cartridge 150 and the extent to which the forward end 122 of the plunger rod 110 extends past the forward end of the cartridge 150 begins to reduce.

At a point during insertion, further forward movement of the cartridge 150 within the housing may be prevented. For example, this may be caused by the cartridge 150 abutting the syringe. In alternative arrangements, the cartridge 150 may engage alternative components (or features on the housing of the auto-injector) such that further forward movement is prevented.

Continued application of the axial force on the actuator 104 when forward movement of the cartridge 150 is prevented may cause relative rotation between the cartridge 150 and the actuator 104. In the exemplary arrangement of Figures 4a and 4b, the relative rotation may be caused by the corresponding feature 170 of the cartridge 150 travelling along the ramped surface 168 of the actuator 104.

In exemplary arrangements, rotation of the cartridge 150 relative to the actuator 104 may cause the cartridge 150 to couple to the rearward portion 108. The relative rotation between the cartridge 150 and the actuator 104 may cause relative rotation between the cartridge 150 and the rearward portion 108 (which is substantially rotationally stationary when decoupled from the cartridge 150). In such arrangements, the rearward portion 108 locking features may engage the corresponding locking features on the cartridge 150 under relative rotation of the cartridge 150 and the rearward portion 108. As described above, in alternative arrangements, the rearward portion 108 may already be coupled to the cartridge 150, in which case rotation of the cartridge 150 may cause corresponding rotation of the rearward portion 108.

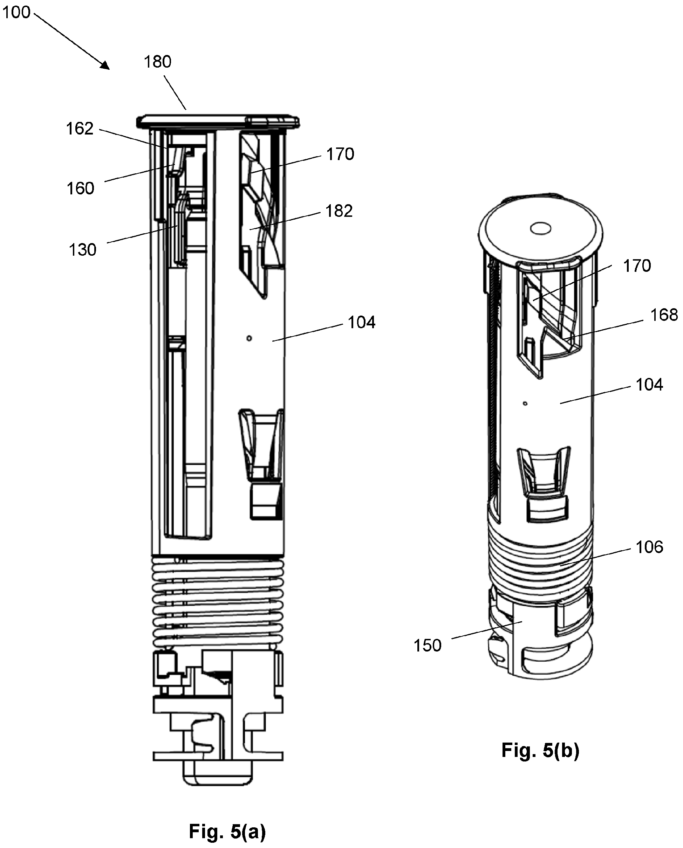

Further rotation of the cartridge 150 relative to the actuator 104 after the cartridge 150 and the rearward portion 108 have coupled causes relative rotation of the retainer 114 with respect to the actuator 104. The relative rotation between the retainer 114 and the actuator 104 may cause the retainer 114 to engage the axial lock 162. In the exemplary arrangement of Figures 4a-4b, the relative rotation between the retainer 114 and the actuator causes the retaining feature 160 of the retainer 114 to engage one of the plurality of locking features of the axial lock 162 of the actuator 104. This is shown in Figures 5a and 5b.

Engagement of the retainer 114 with the axial lock 162 may axially fix the position of the retainer 114 relative to the auto-injector. In exemplary arrangements, the retainer 114 may be configured to engage the axial lock 162 when the apparatus 100 has been fully inserted into the housing of the auto-injector. This may be when the end cap 180 of the actuator 104 abuts a surface of the housing. Abutment between the end cap 180 and the surface of the housing may fix the axial position of the actuator 104 relative to the housing. Since, as described above, the engagement between the retainer 114 and the axial lock 162 fixes the axial position of the retainer 114 relative to the actuator 104, when the apparatus is fully inserted within the housing, the axial position of the retainer 114 relative to the housing may be fixed.

The skilled person will appreciate that the retainer 114 engages the axial lock 162 when the forward end 122 of the plunger rod 110 is at a particular position relative to the bung within the barrel of the syringe. In some arrangements, this particular position may correspond to the forward end 112 of the plunger rod 110 contacting the bung. In alternative arrangements, a separation between the bung and the forward end 122 of the plunger rod 110 may be created during the insertion of the apparatus 100. For example, the firing assembly 102 may be moved rearwards during insertion of the firing assembly within the housing of the auto-injector. In such arrangements, the particular position may correspond to a particular separation between the bung and the forward end of the plunger rod 110.

Figures 5a and 5b show the apparatus at the point at which the retainer 114 engages the axial lock 162. In exemplary arrangements, at this point the travel of the corresponding portion 170 along the angled surface 168 of the actuator 104 has been completed, and the corresponding feature 170 of the cartridge 150 has entered the drive track 182. As shown in Figures 5a and 5b, the corresponding feature 170 may be positioned at the rearmost point of the drive track 182. The corresponding feature 170 may be configured to travel forwards within the drive track 182 under the force of the insertion driver 106, as described below.

On actuation of the auto-injector by a user, the insertion driver 106 may drive one or more of the components of the firing assembly 102 forwards on an insertion stroke. In the exemplary arrangement shown in Figures 4a-5b, the insertion driver biases the cartridge 150 forwards. This causes the corresponding feature 170 to travel along the drive track 182. Since, as described above, the axial position of the actuator 104 is fixed relative to the housing, rearward movement of the actuator 104 under the bias of the insertion driver is prevented.

As described above, the rearward portion 108 is coupled to the cartridge 150. As such, forward movement of the cartridge 150 causes forward movement of the rearward portion 108. This in turn, causes forward movement of the plunger rod 110 and the plunger rod driver 112. Because the axial position of the retainer 114 is fixed relative to the housing, the retainer does not move forwards under the bias of the insertion driver 106. As such, the rearward portion 108, plunger rod 110 and plunger rod driver 112 move axially relative to the retainer 114 on the insertion stroke.

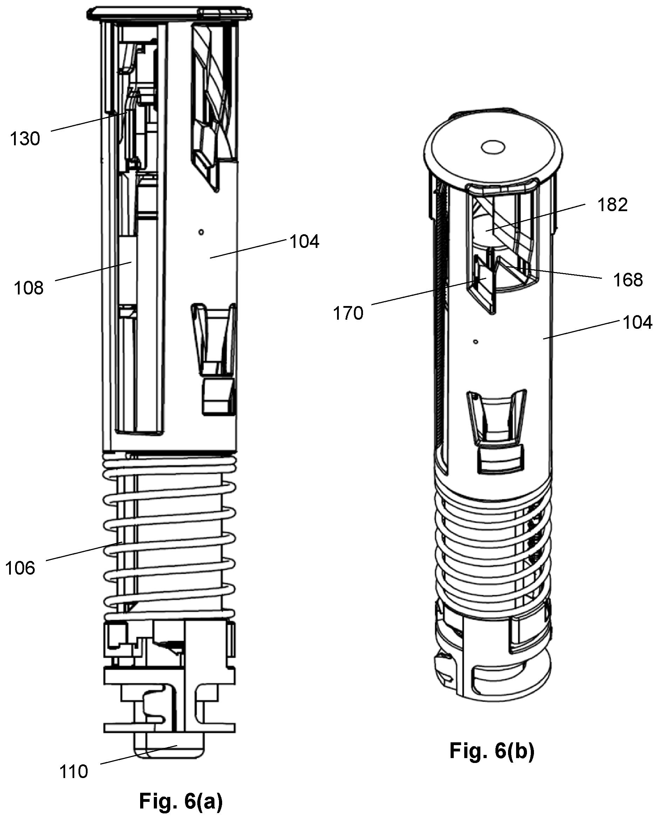

In the exemplary apparatus 100, the relative axial movement disengages the retainer 114 from the plunger rod 110 and the rearward portion 108. In the examples shown, the relative axial movement draws the prong 130 of the retainer 114 out of engagement with the plunger rod 110 and the rearward portion 108. This is shown in Figures 6a and 6b.

At a point on the insertion stroke, the prong 130 is drawn fully out of engagement with the plunger rod 110 and the rearward portion 108. That is, the prong 130 may be drawn fully out of abutment with the surfaces 132 and 134 of the plunger rod 110 and rearward portion 108 respectively. As described above, the point on the insertion stroke at which the prong 130 is drawn fully out of engagement with the plunger rod 110 and the rearward portion 108 may be defined by the axial length of the prong 130. This point may be substantially at the end of the insertion stroke. The end of the insertion stroke may be the point at which the needle of the syringe of the auto-injector is fully inserted into an injection site. At the end of the insertion stroke, in exemplary arrangements, the cartridge 150 may have completed its forward movement, and the corresponding features 170 of the cartridge 150 may be located at the forwardmost point of the driver track 182.

Disengagement of the retainer 114 from the plunger rod 110 and the rearward portion 108 allows forward movement of the plunger rod 110 relative to the rearward portion 108 under the force of the plunger rod driver 112. In the exemplary arrangement shown in Figures 4a-6b disengagement of the retainer 114 allows the plunger rod 110 to rotate relative to the rearward portion 108. This is because the prong 130 of the retainer 114 is no longer preventing travel of the corresponding feature 126 on the plunger rod 110 along the angled surface 124 of the rearward portion 108 under the bias of the plunger rod driver 112. As such, the corresponding feature 126 on the plunger rod 110 travels along the angled surface 124 of the rearward portion 108.

Once the corresponding feature 126 on the plunger rod 110 completes its travel along the angled surface 124 of the rearward portion 108, the plunger rod 110 may be free to travel axially forwards relative to the rearward portion 108 under the force of the plunger rod driver 112. The travel of the plunger rod 110 axially forwards relative to the rearward portion 108 may cause the plunger rod 110 to drive the bung of the syringe within the barrel to dispense a substance contained therein.

It is noted that many of the features of the exemplary apparatus described above and shown in the drawings may be included in other exemplary apparatus. As such, the different drawings are not necessarily to be considered as separate embodiments and features from one drawing may be transferred to an apparatus in another drawing. The skilled person will be able to envisage other apparatus and features thereof without departing from the scope of the appended claims.

The invention can also be described by the following numbered clauses:
Clause 1. An apparatus for use with an auto-injector configured to receive a syringe, the apparatus comprising:
   a firing assembly comprising a rearward portion, a plunger rod moveable forward relative to the rearward portion and a plunger rod driver configured to drive the plunger rod forward relative to the rearward portion;
   an insertion driver configured to drive the syringe forwards on an insertion stroke for inserting a needle of the syringe into an injection site; and
   wherein the firing assembly further comprises a retainer engaged with the plunger rod and/or the rearward portion and configured to prevent forward movement of the plunger rod relative to the rearward portion, and to disengage from the firing assembly at a point on the insertion stroke.
Clause 2. An apparatus according to clause 1, wherein the insertion driver is further configured to drive at least the plunger rod, the plunger rod driver and the rearward portion forwards on the insertion stroke.
Clause 3. An apparatus according to clause 1 or 2, wherein the disengagement of the retainer is caused by relative axial movement between the retainer and the plunger rod, the plunger rod driver and the rearward portion.
Clause 4. An apparatus according to any preceding clause, wherein the retainer is axially fixed relative to a housing of the auto-injector.
Clause 5. An apparatus according to any preceding clause, wherein the plunger rod driver biases the plunger rod forwards relative to the rearward portion.
Clause 6. An apparatus according to clause 5, wherein one of the rearward portion and the plunger rod comprises an angled surface configured to interact with a corresponding feature on the other of the rearward portion and plunger rod under the bias of the plunger rod driver for relative rotation between the rearward portion and plunger rod,
   and wherein the retainer is configured to prevent relative rotation between the rearward portion and plunger rod.
Clause 7. An apparatus according to clause 5 or 6, wherein the retainer comprises a prong configured to abut a surface of the plunger rod to prevent relative rotation between the rearward portion and plunger rod.
Clause 8. An apparatus according to clause 7, wherein forward movement of the firing assembly on the insertion stroke draws the prong out of abutment with the surface of the plunger rod.
Clause 9. An apparatus according to clause 7 or 8, wherein the axial length of the prong defines the point on the insertion stroke at which the retainer disengages from the firing assembly.
Clause 10. An apparatus according to any preceding clause, wherein the point on the insertion stroke at which the retainer disengages from the firing assembly is at substantially the end of the insertion stroke.
clause 11. An auto-injector sub-assembly comprising an apparatus and a housing, the auto-injector sub-assembly comprising:
   a firing assembly comprising a rearward portion, a plunger rod moveable forward relative to the rearward portion and a plunger rod driver configured to drive the plunger rod forward relative to the rearward portion, the firing assembly insertable within the housing;
   an insertion driver configured to drive the syringe forwards on an insertion stroke for inserting a needle of the syringe into an injection site; and
   wherein the firing assembly further comprises a retainer engaged with the plunger rod and/or the rearward portion and configured to prevent forward movement of the plunger rod relative to the rearward portion, and to disengage from the firing assembly at a point on the insertion stroke.

## Claims

1. An apparatus for use with an auto-injector configured to receive a syringe, the apparatus comprising:
a firing assembly comprising a rearward portion, a plunger rod moveable forward relative to the rearward portion, a plunger rod driver configured to drive the plunger rod forward relative to the rearward portion and a retainer engaged with the plunger rod and/or the rearward portion to prevent forward movement of the plunger rod relative to the rearward portion,
wherein the firing assembly is configured for insertion into the auto-injector such that the plunger rod is received within a barrel of the syringe,
and wherein the retainer is configured to engage an axial lock for axially fixing a position of the retainer relative to the auto-injector when a forward end of the plunger rod is at a particular position relative to a bung located within the barrel.

2. An apparatus according to claim 1, further comprising an actuator coupled to the firing assembly and configured to be received in a housing of the auto-injector for insertion of the firing assembly into the auto-injector, wherein the actuator comprises the axial lock.

3. An apparatus according to claim 2, wherein the retainer is configured to engage the axial lock on relative rotation between the actuator and the retainer.

4. An apparatus according to claim 3, wherein the actuator comprises a plurality of locking features, and wherein relative rotation between the actuator and the retainer causes engagement between a retaining feature of the retainer and one of the plurality of locking features.

5. An apparatus according to claim 3 or 4, wherein one of the actuator and the firing assembly comprises a ramped surface configured to interact with a corresponding feature on the other of the actuator and firing assembly to cause the relative rotation between the retainer and the actuator.

6. An apparatus according to any of claims 2 to 5, wherein the actuator comprises a sleeve configured to receive the firing assembly therein.

7. An apparatus according to any of claims 2 to 6, wherein the actuator further comprises an end cap configured to abut a surface of the housing to fix the axial position of the actuator relative to the housing.

8. An apparatus according to claim 7, wherein the retainer is configured to engage the axial lock when the end cap abuts the surface of the auto-injector.

9. An apparatus according to any of claims 1 to 8, further comprising an insertion driver configured to drive the syringe, the plunger rod, the plunger rod driver and the rearward portion forwards on an insertion stroke for inserting a needle of the syringe into an injection site.

10. An apparatus according to claim 9, wherein the axial lock is configured to prevent forward movement of the retainer such that the forward movement of the syringe, the plunger rod, the plunger rod driver and the rearward portion forwards on the insertion stroke disengages the retainer from the plunger rod and/or the rearward portion.

11. An auto-injector sub-assembly comprising an apparatus according to any of claims 1 to 10, and further comprising a housing configured to receive the apparatus.

12. An auto-injector comprising the apparatus of any of claims 1 to 10, and/or the auto-injector sub-assembly of claim 11.

13. An auto-injector according to claim 12 further comprising a syringe comprising a barrel and a bung located therein.
